# EUROPÄISCHE PATENTSCHRIFT

(11) **EP 2 656 768 B1**
(45) Veröffentlichungstag und Bekanntmachung des Hinweises auf die Patenterteilung: **08.06.2016**
(21) Anmeldenummer: 12401073.7
(22) Anmeldetag: 27.04.2012
(51) Int. Cl.: A61B 90/70

(54) **Reinigungs- und Desinfektionsautomat**
Cleaning and disinfection machine
Automate de nettoyage et de désinfection

(43) Veröffentlichungstag der Anmeldung: 30.10.2013
(73) Patentinhaber: Miele & Cie. KG, 33332 Gütersloh (DE)
(72) Erfinder: Heitmann, Michael, 33739 Bielefeld (DE)

(56) Entgegenhaltungen:
- US-A- 4 134 414
- US-A- 5 427 129
- US-A- 5 752 533

## Beschreibung

Die Erfindung betrifft einen Reinigungs- und Desinfektionsautomat insbesondere für medizinische Instrumente und Geräte, mit einem einen Spülraum bereitstellenden Spülbehälter, der in einen Sammeltopf mündet, einer im Spülraum angeordneten Sprüheinrichtung zur Beschickung von Spülgut mit Spülflotte und einer der Umwälzung der Spülflotte dienenden Umwälzpumpe.

Reinigungs- und Desinfektionsautomaten der vorbeschriebenen Art sind aus dem Stand der Technik an sich gut bekannt. Sie verfügen über einen Spülbehälter, der einen Spülraum bereitstellt. Im bestimmungsgemäßen Verwendungsfall dient der Spülraum der Aufnahme von zu reinigendem und/oder zu desinfizierendem Spülgut, bei dem es sich beispielsweise um medizinische Instrumente und Geräte handeln kann. Der Spülraum ist über eine Beschickungsöffnung des Spülbehälters zugänglich, die mittels einer verschwenkbar am Spülbehälter angeordneten Tür fluiddicht verschließbar ist.

Typischerweise kommt im bestimmungsgemäßen Verwendungsfall zur Spülgutreinigung ein Spülkorb zum Einsatz. Dieser wird mit dem zu reinigenden Spülgut bestückt und anschließend verwenderseitig durch die Beschickungsöffnung in den Spülraum eingeführt. Nach erfolgreich durchgeführter Spülung, d. h. Reinigung und/oder Desinfektion des Spülguts kann der Spülkorb zusammen mit den davon aufgenommenen Spülgütern verwenderseitig dem Spülraum durch die Beschickungsöffnung wieder entnommen werden.

Innerhalb des Spülraums ist eine Sprüheinrichtung angeordnet. Diese dient der Beschickung von Spülgut mit Spülflotte. Typischerweise weist die Sprüheinrichtung Sprüharme auf, die verdrehbar gelagert ausgebildet sind. Es kann dabei zwischen automatenseitigen Sprüharmen einerseits und korbseitigen Sprüharmen andererseits unterschieden werden. Dabei betreffen "automatenseitige Sprüharme" solche Sprüharme, die im Spülraum des Automaten installiert sind. In der Regel sind zwei solcher Sprüharme vorgesehen, wobei der eine deckenseitig und der andere bodenseitig des Spülraums ausgebildet ist. "Korbseitige Sprüharme" betreffen indes solche Sprüharme, die am Spülkorb angeordnet sind, die also zusammen mit dem Spülkorb verwenderseitig verfahren werden können. Zum Anschluss dieser Sprüharme an ein Wasser- und/oder Spülflottenzuführsystem sind im Spülraum entsprechend ausgebildete Ankopplungsstellen vorgesehen, wie beispielsweise aus der DE 10 2007 003 894 A1 bekannt.

Die Sprüheinrichtung kann je nach Ausgestaltung des Reinigungs- und Deisinfektionsautomaten nicht nur der Abgabe von Spülflotte dienen. Es sind Ausgestaltungsformen bekannt geworden, wonach die Sprüheinrichtung auch dazu dient, das Spülgut nach erfolgter Reinigung und/oder Desinfektion zu trocknen, zu welchem Zweck Trocknungsluft in den Spülraum über die Sprüheinrichtung eingeleitet wird. In diesem Fall dient die Sprüheinrichtung einerseits dazu, während des eigentlichen Reinigungs- und/oder Desinfektionsvorganges Spülflotte auf das zu reinigende und/oder zu desinfizierende Spülgut abzugeben sowie andererseits dazu, nach Abschluss eines solchen Reinigungs- und/oder Desinfektionsvorganges das Spülgut mit Trocknungsluft zu beaufschlagen.

Um über die Sprüheinrichtung die Abgabe einerseits von Spülflotte und andererseits von Trocknungsluft zu gestatten, kommen bei Reinigungs- und Desinfektionsautomaten kleinerer Bauart entsprechende Trenn- und Verteileinrichtungen zum Einsatz. Diese sorgen für eine Beschickung der Sprüheinrichtung entweder mit Spülflotte oder mit Trocknungsluft.

Aus dem Stand der Technik sind auch Konstruktionen bekannt geworden, die nicht über Trenn- und Verteileinrichtungen der vorbeschriebenen Art verfügen. Bei solchen Automaten handelt es sich typischerweise um Großgeräte, die über eine Mehrzahl von Sprüharmen oder dergleichen Fluidabgabeeinrichtungen verfügen, die typischerweise in Höhenrichtung übereinander angeordnet sind. Zur Versorgung dieser Sprüharme entweder mit Spülflotte oder mit Trocknungsluft sind jeweils Speiseleitungen vorgesehen, die über außerhalb des Spülraums ausgebildete Verteileinrichtungen strömungstechnisch miteinander koppelbar bzw. voneinander entkoppelbar sind. Ein solcher Aufbau ist vergleichsweise kompliziert und störanfällig und hat aus diesem Grunde bislang nur bei Großgeräten Einsatz gefunden.

Sowohl bei Reinigungs- und Desinfektionsautomaten kleinerer Bauart als auch bei Großgeräten kommt zum Zwecke der Umwälzung von Spülflotte eine Umwälzpumpe zum Einsatz. Über diese wird im Spülraum befindliche Spülflotte angesaugt und über entsprechende Versorgungsleitungen Sprüharmen oder sonstigen Fluidabgabeeinrichtungen der Sprüheinrichtung zugeführt. Diese Versorgungsleitungen sind zur Maximierung der Größe des nutzbaren Spülraums außerhalb des Spülraums verlegt, wie sich dies beispielsweise aus der DE 10 2009 009 768 A1 und der DE 10 2006 009 787 A1 ergibt. Die Anordnung der Versorgungsleitungen außerhalb des Spülraums ist darüber hinaus mit Blick auf die schon vorbeschriebene Ankopplung von korbseitigen Sprüharmen an ein Fluidzuführungssystem von Vorteil.
Die US 5427129 A und die US5377707A offenbaren einen Geschirrspüler, bei dem der Sammeltopf der Umwälzpumpe als Pumpengehäuse dient.

Obgleich sich die vorbeschriebene Konstruktion im alltäglichen Praxiseinsatz bewährt hat, besteht Verbesserungsbedarf, insbesondere mit Blick auf eine noch weiter vereinfachte Handhabung.

Es ist deshalb ausgehend von dem Vorbeschriebenen die **Aufgabe** der Erfindung, einen neuartigen Reinigungs- und Desinfektionsautomaten vorzuschlagen, der bei gleichzeitiger Verbesserung der Betriebssicherheit eine vereinfachte Handhabung gestattet.

Zur **Lösung** dieser Aufgabe wird mit der Erfindung ein Reinigungs- und Desinfektionsautomat mit den Merkmalen von Anspruch 1 vorgeschlagen.

Bei den aus dem Stand der Technik bekannten Konstruktionen stellen die Umwälzpumpe einerseits und der Sammeltopf andererseits zwei voneinander getrennte Bauteile dar. Zum Zwecke der strömungstechnischen Verbindung sind entsprechende Wasserleitungen vorgesehen, die konstruktionsbedingt Umlenkungen aufweist. Die Versorgung der Sprüheinrichtung erfolgt ausgehend von der Umwälzpumpe ebenfalls über Versorgungsleitungen, die außerhalb des Spülraums geführt sind und konstruktionsbedingt ebenfalls über entsprechende Umlenkungen verfügen.

Dieses vorbekannte Konstruktionsprinzip erweist sich insofern als nachteilig, als das die Umlenkungen verlustbehaftet sind. Darüber hinaus findet über die zahlreichen Versorgungsleitungen eine nicht unerhebliche Bindung von Spülflotte statt, was im Ergebnis zu einem erhöhten Verbrauch an Spülflotte, d. h. insbesondere Frischwasser führt. Ferner stellen insbesondere die außerhalb des Spülraums verlegten Versorgungsleitungen eine Vielzahl von potentiellen Leckstellen bereit, wodurch eine Gefährdung der elektrischen Verbraucher und Verbindungen gegeben ist. Die verzweigte Spülflottenführung erschwert ferner eine optimierte Spülflotten- bzw. Restwasserentleerung und macht aufwendige Bypass-Spülungen zur Desinfektion erforderlich.

Mit der erfindungsgemäßen Ausgestaltung wird nun eine völlig andere Konstruktion vorgeschlagen. Nach dem erfindungsgemäßen Konzept sind der Sammeltopf und das Pumpengehäuse der Umwälzpumpe als ein Bauteil ausgebildet, d. h. der Sammeltopf dient der Umwälzpumpe als Pumpengehäuse. In der Konsequenz kann auf Versorgungsleitungen, wie sie bei aus dem Stand der Technik bekannten Konstruktionen von Nöten sind, vollends verzichtet werden, womit der erfindungsgemäßen Konstruktion die vorbeschriebene Nachteile nicht anhaften.

Die Ausgestaltung des Sammeltopfes als Pumpengehäuse erlaubt eine Ausführungsform der Erfindung, bei der die Umwälzpumpe im Vergleich zu den aus dem Stand der Technik bekannten Konstruktionen um 90° verdreht ausgerichtet ist, d. h. das Pumpenrad der Umwälzpumpe und die im Spülraum angeordneten Sprüharme der Sprüheinrichtung um ein und dieselbe Drehachse drehen. Hierdurch wird ein besonders strömungsoptimierter Wasserweg realisiert. Eine konventionelle Ausrichtung der Umwälzpumpe, wie sie aus dem Stand der Technik bekannt ist, bei der die Drehachse des Pumpenrades einerseits und die Drehachse der Sprüharme andererseits quer zueinander ausgerichtet sind, in der Regel unter Ausbildung eines 90°-Winkels, kann grundsätzlich auch eingesetzt werden.

Die erfindungsgemäße Ausgestaltung beschreitet jedoch insofern einen völlig anderen Lösungsweg, als das gemäß dieser vorgeschlagen wird, Pumpenrad und Sprüharme um ein und dieselbe Drehachse verdrehbar auszubilden. Dabei ist es bevorzugt, dass die Mittelachse des Spülraums und die Drehachse des Pumpenrads und der Sprüharme zusammenfallen.

Zur Versorgung der Sprüheinrichtung mit Spülflotte dient ein Versorgungsrohr, das sich innerhalb des Spülraums entlang der Drehachse der Sprüharme und des Pumpenrads erstreckt. An dieses Versorgungsrohr ist einendseitig die Umwälzpumpe angeschlossen, so dass von dieser umgewälzte Spülflotte direkt in das Versorgungsrohr gefördert wird, über welches dann eine Verteilung in die einzelnen Sprüharme oder sonstige Fluidabgabeeinrichtungen der Sprüheinrichtung stattfindet. Das Versorgungsrohr ist im Unterschied zu den aus dem Stand der Technik bekannten Versorgungsleitungen nicht außerhalb des Spülraums, sondern vielmehr innerhalb desselben angeordnet. Das Risiko von Leckagen wird so auf ein Minimum reduziert. Darüber hinaus werden im Unterschied zum Stand der Technik die Wasser- bzw. Spülflottenwege erheblich verkürzt, womit einerseits eine vereinfachte Restwasserentleerung stattfinden kann und andererseits Bypass-Spülungen zur Selbstdesinfektion auf Grund des Wegfalls verzweigter Wasser- bzw. Spülflottenwege erheblich minimiert werden können.

Das Pumpenrad der Umwälzpumpe wirkt mit einem fest- bzw. stillstehenden Leitrad zusammen. Die im Betriebsfall am Umfang des Pumpenrades austretende Wassermenge wird durch dieses Leitrad aufgefangen und zur Drehachse hin umgelenkt, so dass sie ohne weitere Umwege und Umlenkungen in Richtung der Drehachse in das im Innenraum des Automaten angeordnete Versorgungsrohr eingeleitet werden kann.

An der tiefsten Stelle des Sammeltopfes bzw. des Pumpengehäuses ist bevorzugter Weise ein Ablaufstutzen vorgesehen. Über diesen kann eine Spülflotten- bzw. Restwasserentleerung stattfinden, was im Unterschied zum Stand der Technik mangels aufwendiger Verzweigungswege sehr viel einfacher und besser erfolgen kann. In diesem Zusammenhang ist es ferner bevorzugt, das den Sammeltopf bzw. das Pumpengehäuse gegenüber dem weiteren Spülraum begrenzende Flächensieb mit einem Reservoire direkt vor dem Ablaufstutzen auszurüsten, in welchem sich während eines bestimmungsgemäßen Spülprogramms gröbere Partikel, Verschmutzungen und/oder dergleichen ansammeln können, die nicht durch das Flächensieb hindurchgeführt werden können, so dass sie bei einem Spülflottenablauf über den Ablaufstutzen mit weggespült werden.

Die das Pumpenrad antreibende Pumpenwelle ist als Hohlwelle ausgebildet. Dies gestattet es, die Sprüharme über eine Antriebswelle anzutreiben, die die Hohlwelle durchragt, womit eine sehr kompakte Ausgestaltung erreicht ist. Dabei hat ein gesonderter Antrieb der Sprüharme den Vorteil, dass sich über eine Rückmeldung des elektrischen Antriebes Aussagen über Drehzahl und Blockierungsfreiheit der Sprüharme ableiten lassen, was wiederum in einer Steuerung ausgewertet werden kann. Ein vorbestimmter und nachsteuerbarer Drehbetrieb der Sprüharme ist damit gestattet.

Die Sprüheinrichtung nach der Erfindung kann auch zur Beaufschlagung des Spülguts mit Trocknungsluft genutzt werden. Zu diesem Zweck ist das zentral im Spülraum angeordnete Versorgungsrohr anderendseitig an ein Gebläse angekoppelt. Die vom Gebläse erzeugte Trocknungsluft wird in das Versorgungsrohr eingeleitet, von wo aus die einzelnen Sprüharme und/oder sonstigen Fluidabgabeeinrichtungen der Sprüheinrichtung mit Trocknungsluft versorgt werden.

Zur strömungstechnischen Trennung von Versorgungsrohr einerseits und Umwälzpumpe andererseits im Falle der Beaufschlagung des Versorgungsrohr mit Trocknungsluft kommt bevorzugter Weise eine Ventileinrichtung zum Einsatz, die beispielsweise vom Leitrad der Umwälzpumpe getragen ist. Diese Ventileinrichtung verfügt über eine Mehrzahl von verschwenkbaren Segmenten, die in geöffneter Stellung die Umwälzpumpe strömungstechnisch mit dem Versorgungsrohr koppeln und in geschlossener Stellung für eine Trennung dieser strömungstechnischen Verbindung sorgen. Dabei ist es bevorzugt, dass die Segmente bei eingeschalteter Umwälzpumpe aufgrund des geförderten Volumenstroms automatisch öffnen, bei ausgeschalteter Umwälzpumpe, d. h. während eines Trocknungsvorganges auf Grund der auf sie einwirkenden Schwerkraft bzw. auf Grund der entgegengesetzt in das Versorgungsrohr einströmenden Trocknungsluft automatisch schließen.

Die erfindungsgemäße Konstruktion erbringt gegenüber dem Stand der Technik insgesamt insbesondere folgende Vorteile:
- Die Länge der Wasserwege und die Anzahl der Umlenkungen ist deutlich reduziert, womit eine Minimierung der Strömungsverluste erreicht ist. Gemäß der vorgeschlagenen Konstruktion fördert die Umwälzpumpe direkt in Richtung der zentralen Spülflottenführung in den Spülraum hinein.
- Gegenüber dem Stand der Technik ist eine deutliche Reduzierung des Volumens für die Wasserführung erreicht, womit die in den Führungswegen gebundene Wassermenge mit dem Vorteil reduziert ist, dass bei bestimmungsgemäßer Verwendung eine gegenüber dem Stand der Technik verringerte Menge an Frischwasser erforderlich ist.
- Die Anzahl möglicher Leckagestellen ist deutlich verringert, was die Betriebssicherheit insgesamt erhöht.
- Die Anzahl der eingesetzten Bauteile und Baukomponenten ist deutlich reduziert, was nicht zuletzt eine Kostenreduktion in der Herstellung und Wartung erbringt.
- Auf außerhalb des Spülraums verlegte Versorgungsleitungen wird vollständig verzichtet. Dies gestattet es, den hierdurch frei werdenden Bauraum für eine Spülraumvergrößerung zu nutzen. Im Ergebnis erbringt dies eine Kapazitätserweiterung, was ebenfalls dazu beiträgt, Ressourcen schonen zu können.
- Das Spülraumunterteil ist im Unterschied zum Stand der Technik fertigungstechnisch deutlich vereinfacht, da es als Prägeteil und nicht mehr als Tiefziehteil mit angebrachtem Schweißteil ausgebildet werden kann, was die Herstellung insgesamt vereinfacht und kostengünstiger gestaltet.
- Der Restwasser- bzw. Spülflottenablauf ist erheblich verbessert, da auf verzweigte, externe Wasserwege konstruktiv verzichtet ist, eine Entleerung solcher Wasserwege im Unterschied zum Stand der Technik also nicht weiter erforderlich ist.
- Es kann ferner auf Bypass-Spülungen für entlegene Ablaufelemente und -schläuche verzichtet werden, da derartige entlegene Ablaufelemente und -schläuche bei der erfindungsgemäßen Konstruktion nicht mehr vorhanden sind.

Es ergibt sich damit alles in allem eine gegenüber dem Stand der Technik deutlich vereinfachte Handhabung.

Weitere Vorteile und Merkmale der Erfindung ergeben sich aus der nachfolgenden Beschreibung anhand der Figuren.

Dabei zeigen
- Figur 1: in einer schematischen Ansicht einen Reinigungs- und Desinfektionsautomaten nach der Erfindung;
- Figur 2: in einer schematischen Darstellung insbesondere den Spülbehälter eines erfindungsgemäßen Reinigungs- und Desinfektionsautomaten;
- Figur 3: in einer schematischen Ausschnittdarstellung den unteren Teil des Spülbehälters nach Figur 2;
- Figur 4: in schematisch perspektivischer Darstellung eine nach der Erfindung vorgesehenen Ventileinrichtung in geöffneter Stellung und
- Figur 5: in schematischer Darstellung die Ventileinrichtung nach Figur 4 in geschlossener Stellung.

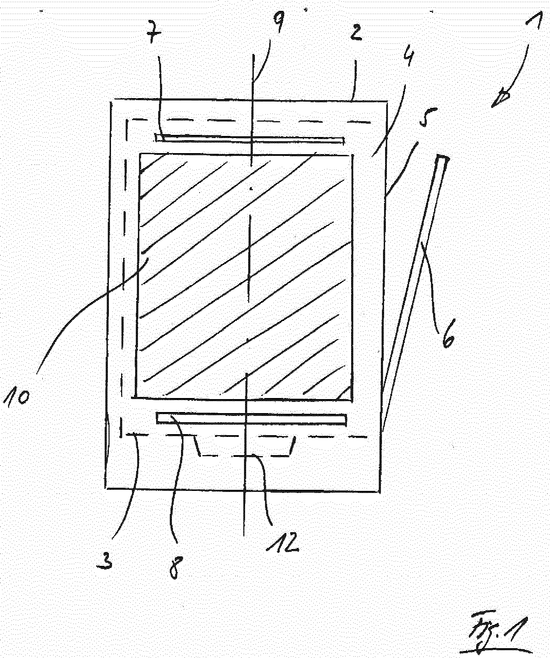

Figur 1 lässt in schematischer Seitenansicht einen Reinigungs- und Desinfektionsautomaten nach der Erfindung erkennen, im Nachfolgenden Spülautomat 1 genannt.

Der Spülautomat 1 nach der Erfindung ist als Gewerbeautomat ausgelegt und dient insbesondere der Behandlung von medizinischen Instrumenten und Geräten, beispielsweise Glaskolben, Reagenzgläsern, Schläuchen und/oder dergleichen.

Der Spülautomat 1 verfügt über ein Gehäuse 2. Innerhalb des Gehäuses 2 ist ein Spülbehälter 3 angeordnet, der einen Spülraum 4 bereitstellt. Der Spülraum 4 ist über eine Beschickungsöffnung 5 zugänglich, welche Beschickungsöffnung 5 mittels einer Spülraumtür 6 fluiddicht verschließbar ist.

Im bestimmungsgemäßen Verwendungsfall dient der Spülraum 4 der Aufnahme von zu reinigendem Spülgut, beispielsweise medizinischen Instrumenten und Geräten. Zur Positionierung des zu reinigenden Spülgutes innerhalb des Spülraums 4 dient ein Spülkorb 10. Dieser kann verwenderseitig aus dem Spülraum 4 herausverfahren bzw. in diesen eingebracht werden.

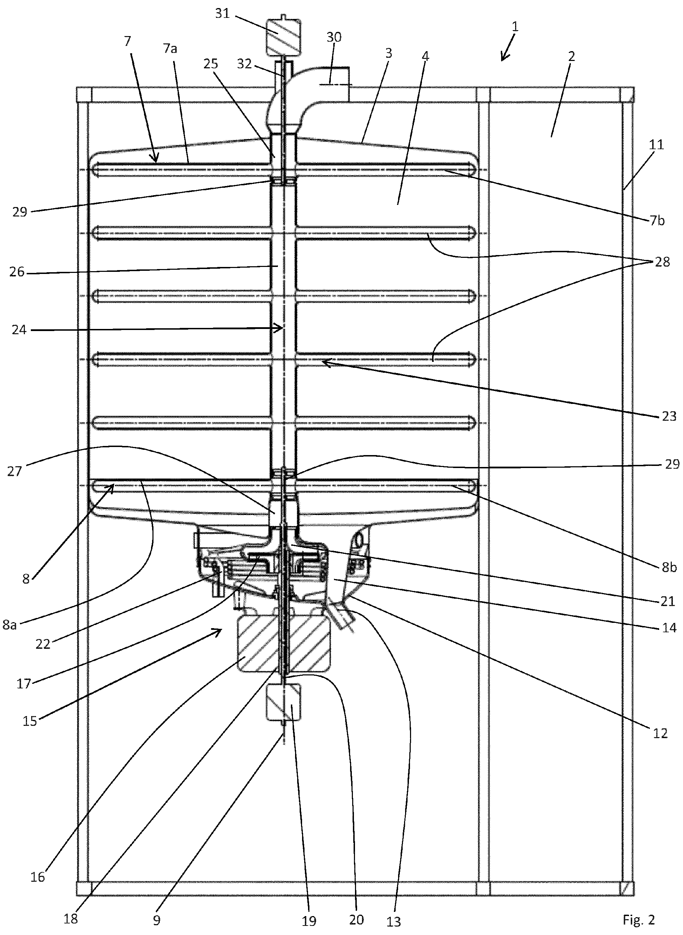

Zum Zwecke der Beschickung des Spülraums 4 mit Spülflotte oder mit Trocknungsluft kommt eine Sprüheinrichtung 23 zum Einsatz, wie sie sich insbesondere aus der Darstellung nach Figur 2 ergibt. Die Sprüheinrichtung 23 verfügt einerseits über automatenseitige Sprüharme 7 und 8 sowie andererseits über korbseitige Sprüharme 28. Der automatenseitige Sprüharm 7 ist innerhalb des Spülraumes 4 deckenseitig montiert und verfügt über zwei Sprüharmäste 7a und 7b. Der zweite automatenseitige Sprüharm 8 ist in gleicher Weise ausgebildet und verfügt über Sprüharmäste 8a und 8b. Dabei ist der Sprüharm 8 bodenseitig des Spülraums 4 ausgebildet. Beide Sprüharme 7 und 8 drehen um die gemeinsame Drehachse 9.

Der Spülraum 4 mündet bodenseitig in einen Sammeltopf 12 ein, wie dies insbesondere der schematischen Darstellung nach Figur 1 entnommen werden kann.

Figur 2 lässt den Aufbau des erfindungsgemäßen Spülautomaten 1 genauer erkennen. Wie dieser Darstellung zu entnehmen ist, ist der Spülbehälter 3 innerhalb des von einem Gestell 11 getragenen Gehäuses 2 angeordnet. Der Spülbehälter 3 geht bodenseitig in den Sammeltopf 12 über.

Zum Zwecke der Umwälzung der im bestimmungsgemäßen Verwendungsfall im Spülraum 4 befindlichen Spülflotte dient eine Umwälzpumpe 15. Diese saugt die sich im Sammeltopf 12 ansammelnde Spülflotte an und fördert diese zur Sprüheinrichtung 23, von wo aus dann eine Beschickung von Spülgut mit Spülflotte erfolgt.

Nach der erfindungsgemäßen Ausgestaltung ist vorgesehen, dass der Sammeltopf 12 der Umwälzpumpe 15 als Pumpengehäuse dient, zu welchem Zweck der Sammeltopf 12 und das Pumpengehäuse als ein Bauteil ausgebildet sind. Figur 2 lässt diese Ausgestaltung gut erkennen.

Der vom Sammeltopf 12 bzw. dem Pumpengehäuse bereitgestellte Volumenraum ist vom übrigen Spülraum 4 durch ein Flächensieb 14 getrennt. Mittels diesem Flächensieb 14 können grobe Verschmutzungen zurückgehalten werden. Dabei ist es bevorzugt, das Flächensieb 14 mit einem Reservoire 14a auszurüsten, in welchem vom Flächensieb 14 zurückgehaltene Partikel, Verschmutzungen und/oder dergleichen Fremdstoffe während der Durchführung eines Spülprogramms gesammelt werden können.

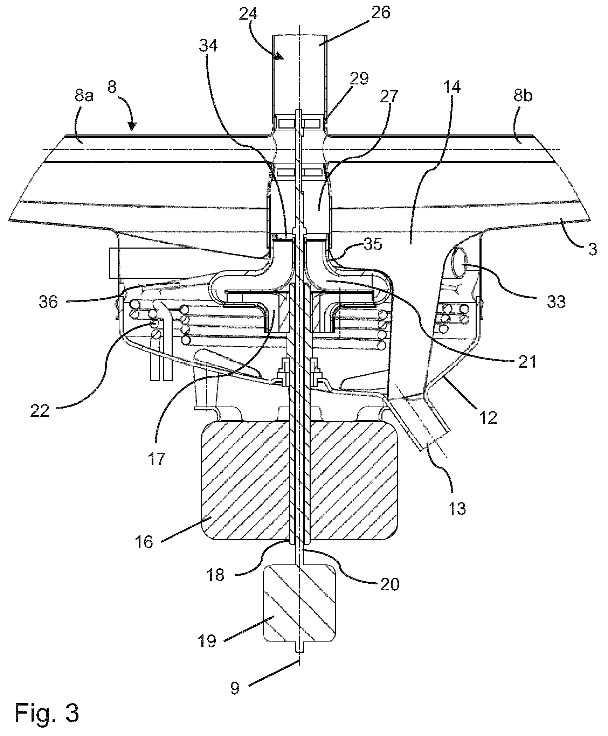

Der Sammeltopf 12 ist mit einem Ablaufstutzen 13 ausgerüstet. Dieser ist an der tiefsten Stelle des Sammeltopfes 12 ausgebildet und dient nach erfolgreich durchlaufenem Spülprogramm der Wasser- bzw. Spülflottenentleerung. Dabei ist es bevorzugt, das Reservoire 14a des Flächensiebs 14 direkt oberhalb des Ablaufstutzens 13 auszugestalten, wie dies in Figur 3 gezeigt ist. Im Reservoire 14a angesammelte Partikel, Verschmutzungen und/oder dergleichen Fremdstoffe können so bei einer Restwasserentleerung direkt über den Ablaufstutzen 13 abgepumpt werden.

Die Umwälzpumpe 15 verfügt über einen außerhalb des Sammeltopfes 12 ausgebildeten Motor 16. Dieser treibt über eine Pumpenwelle 18 das im Sammeltopf 12 angeordnete Pumpenrad 17 an. Dieses wirkt mit einem fest-, d. h. stillstehenden Leitrad 21 zusammen. Die im bestimmungsgemäßen Betriebsfall am Umfang des Pumpenrades 17 austretende Wassermenge wird durch das Leitrad 21 aufgefangen und in Richtung der Drehachse 9 umgelenkt und mit Bezug auf die Zeichnungsebene nach den Figuren 2 und 3 nach oben in das Verteil- oder Versorgungsrohr 24 eingeleitet, von wo aus dann eine Bedienung der Sprüharme 7, 8 und 28 stattfindet.

Im Sammeltopf 12 bzw. im Pumpengehäuse der Umwälzpumpe 15 können ferner Elektro- und/oder Dampfheizkörper in Form einer Heizeinrichtung 22 und/oder einer Dampfheizeinrichtung 33 vorgesehen sein. Damit dient der Sammeltopf 12 nicht nur der Funktion als Ansaugvolumen für die Umwälzpumpe 15, er wird auch für die Unterbringung entsprechender Heizeinrichtungen 22 bzw. 23 genutzt.

Das zur Versorgung der Sprüheinrichtung 23 mit Spülflotte vorgesehene Versorgungsrohr 24 verfügt über insgesamt drei Abschnitte 25, 26 und 27. Dabei dient der erste Abschnitt 25 als oberer Abschnitt, der mit dem deckenseitigen Sprüharm 7 gekoppelt ist. Der zweite Abschnitt 27 dient als unterer Abschnitt, der seinerseits mit dem bodenseitigen Sprüharm 8 gekoppelt ist. Der dritte Abschnitt 26 dient als mittlerer Abschnitt und ist korbseitig, d. h. als Bestandteil des dem Spülraum 4 entnehmbaren Spülkorbs 10 ausgebildet. Die Abschnitte 25, 26 und 27 sind im bestimmungsgemäßen Verwendungsfall fluiddicht miteinander verbunden.

Im einfachsten Ausführungsfall verfügt die Sprüheinrichtung 23 über die beiden automatenseitigen Sprüharme 7 und 8. Im bestimmungsgemäßen Verwendungsfall ist der dritte, d. h. der mittlere Abschnitt 26 des Versorgungsrohrs 24 fluiddicht mit den beiden anderen Abschnitten 25 und 27 verbunden, so dass über die Umwälzpumpe 15 geförderte Spülflotte einerseits den bodenseitigen Sprüharm 8 und über das Versorgungsrohr 24 auch den deckenseitigen Sprüharm 7 erreicht.

Gemäß der in den Figuren gezeigten Ausführungsform sind weitere Sprüharme 28 vorgesehen, die als spülkorbseitige Sprüharme 28 direkt an das Versorgungsrohr 24 angeschlossen sind. Im bestimmungsgemäßen Verwendungsfall findet damit über das Versorgungsrohr 24 nicht nur eine Beschickung der Sprüharme 7 und 8, sondern auch eine Beschickung der mit dem Versorgungsrohr 24 gekoppelten Sprüharme 28 statt.

Zur Kupplung des ersten Abschnitts 25 und des dritten Abschnitts 26 des Versorgungsrohrs 24 bzw. des zweiten Abschnitts 27 und des dritten Abschnitts 26 des Versorgungsrohrs 24 sind jeweils Kupplungsstellen 29 vorgesehen, die für eine fluiddichte Verbindung sorgen. Figur 3 lässt die Kupplungsstelle 29 zwischen zweitem Abschnitt 27 und drittem Abschnitt 26 beispielhaft erkennen.

Gemäß einer bevorzugten Ausführungsform findet ein elektromotorischer Antrieb der Sprüharme 7,8 und 28 der Sprüheinrichtung 23 statt. Zu diesem Zweck ist ein Motor 19 vorgesehen, der über eine Antriebswelle 20 an die verdrehbar gelagerten Sprüharme 7, 8 und 28 angeschlossen ist. Dabei ist es bevorzugt, dass die Verbindung der einzelnen Abschnitte 25, 26 und 27 des Versorgungsrohrs 24 kraftübertragend ausgebildet ist, so dass über einen Antrieb allein des unteren, zweiten Abschnitts 27 des Versorgungsrohrs 24 ein Antrieb sämtlicher Sprüharme 7, 8 und 28 erreicht ist.

Die Antriebswelle 20 erstreckt sich bevorzugter Weise entlang der Drehachse 9, zu welchem Zweck die Pumpenwelle 18 als Hohlwelle ausgebildet ist, durch die hindurch die Antriebswelle 20 zum Antrieb der Sprüharme 7, 8 und 28 ragt.

Gemäß einer alternativen Ausgestaltung der Erfindung kann ein Antrieb der Sprüharme 7, 8 und 28 auch von oben, d. h. deckenseitig erfolgen. Gemäß dieser Alternative sind ein Motor 31 und eine Welle 32 vorgesehen, die auf den oberen, d. h. ersten Abschnitt 25 des Versorgungsrohrs 24 und den damit gekoppelten Sprüharm 7 einwirken. Der Motor 19 und die Antriebswelle 20 können im Falle dieser alternativen Ausgestaltungsform entallen.

Die Sprüheinrichtung 23 dient nicht nur der Beschickung von Spülgut mit Spülflotte, sondern auch der Beschickung des Spülguts mit Trocknungsluft. Zu diesem Zweck ist ein Lufteinlass 30 vorgesehen, der an ein in den Figuren nicht näher dargestelltes Gebläse angeschlossen ist. Über den Lufteinlass 30 kann vom Gebläse geförderte Luft in das Versorgungsrohr 24 geleitet werden, von wo aus dann eine Verteilung auf die einzelnen Sprüharme 7, 8 und 28 stattfindet.

Neben den in den Figuren gezeigten Sprüharmen 7, 8 und 28 kann die Sprüheinrichtung 23 auch andere Fluidabgabeeinrichtungen aufweisen, z. B. in Form von Anschlussstutzen, an die in einfacher Weise beispielsweise Schläuche durch Aufstecken angeschlossen werden können.

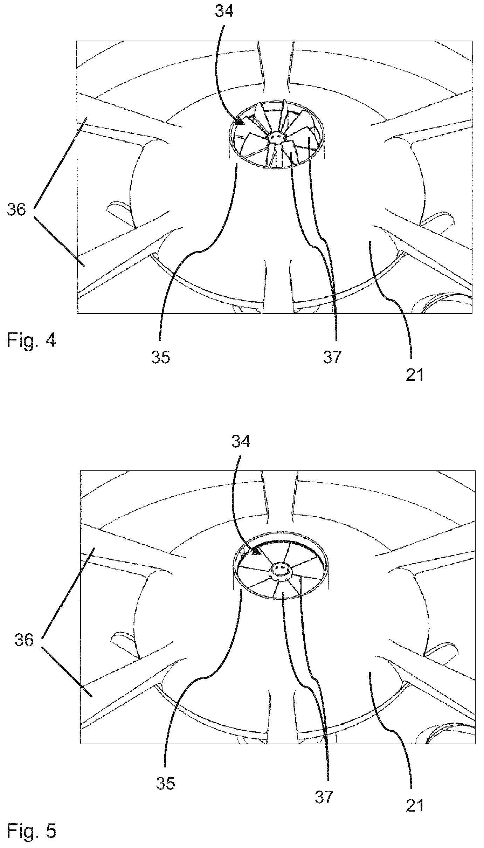

Um im Trocknungsbetrieb eine strömungstechnische Trennung von Umwälzpumpe 15 und Versorgungsrohr 24 zu erreichen, ist eine Ventileinrichtung 34 vorgesehen, wie sie sich aus den Figuren 4 und 5 ergibt. Diese Ventileinrichtung 34 ist von einem Kragen 35 des Leitrads 21 getragen, welches sich über Stege 36 am Sammeltopf 12 abstützt. Wie die Figuren 4 und 5 erkennen lassen, verfügt die Ventileinrichtung 34 über verschwenkbare Segmente 37. Dabei zeigt Figur 4 die geöffnete und Figur 5 die geschlossene Stellung der Ventileinrichtung 34.

Bei eingeschalteter Umwälzpumpe 15 strömt Spülflotte direkt in das im Spülraum 4 ausgebildete Versorgungsrohr 34 ein. In Folge dieser Einströmbewegung werden die Segmente 37 der Ventileinrichtung 24 in ihre geöffnete Stellung gemäß Figur 4 überführt. Bei ausgeschalteter Umwälzpumpe 15 fallen die Segmente 37 der Schwerkraft folgend in ihre geschlossene Stellung gemäß Figur 5 zurück. Gegebenenfalls kann ein Verschwenken der Segmente 37 in die geschlossene Stellung auch durch die in das Versorgungsrohr 24 einströmende Trocknungsluft bewirkt oder unterstütz werden. Jedenfalls sind bei einströmender Trocknungsluft die Segmente 37 in ihre Verschlussstellung überführt, so dass die Umwälzpumpe 15 strömungstechnisch vom Versorgungsrohr 24 getrennt ist.

Die Besonderheit der erfindungsgemäßen Konstruktion liegt darin, dass der Sammeltopf 12 und das Pumpengehäuse der Umwälzpumpe 15 als ein und dasselbe Bauteil ausgebildet sind. Die Drehachse 9 des Pumpenrades 17 der Umwälzpumpe 15 und die Drehachse 9 der Sprüharme 7, 8 und 28 fallen aufgrund dieser konstruktiven Ausgestaltung zusammen. Dabei ist vorgesehen, den von der Umwälzpumpe 15 geförderten Volumenstrom direkt über ein mit der Umwälzpumpe 15 gekoppeltes und im Spülraum 4 angeordnetes Versorgungsrohr 24 in die Sprüheinrichtung 23 einzuleiten.

**Bezugszeichenliste**

| | | | |
|---|---|---|---|
| 1 | Spülautomat | 17 | Pumpenrad |
| 2 | Gehäuse | 18 | Pumpenwelle |
| 3 | Spülbehälter | 19 | Motor |
| 4 | Spülraum | 20 | Antriebswelle |
| 5 | Beschickungsöffnung | 21 | Leitrad |
| 6 | Spülraumtür | 22 | Heizeinrichtung |
| 7 | Sprüharm | 23 | Sprüheinrichtung |
| 7a | Sprüharmast | 24 | Versorgungsrohr |
| 7b | Sprüharmast | 25 | erster Abschnitt |
| 8 | Sprüharm | 26 | dritter Abschnitt |
| 8a | Sprüharmast | 27 | zweiter Abschnitt |
| 8b | Sprüharmast | 28 | Sprüharm |
| 9 | Drehachse | 29 | Kupplungsstelle |
| 10 | Spülkorb | 30 | Lufteinlass |
| 11 | Gestell | 31 | Motor |
| 12 | Sammeltopf | 32 | Welle |
| 13 | Ablaufstutzen | 33 | Dampfheizeinrichtung |
| 14 | Flächensieb | 34 | Ventileinrichtung |
| 14a | Reservoire | 35 | Kragen |
| 15 | Umwälzpumpe | 36 | Steg |
| 16 | Motor | 37 | Segment |

## Patentansprüche

1. Reinigungs- und Desinfektionsautomat insbesondere für medizinische Instrumente und Geräte, mit einem einen Spülraum (4) bereitstellenden Spülbehälter (3), der in einen Sammeltopf (12) mündet, einer im Spülraum (4) angeordneten Sprüheinrichtung (23) zur Beschickung von Spülgut mit Spülflotte und einer der Umwälzung der Spülflotte dienenden Umwälzpumpe (15),wobei der Sammeltopf (12) der Umwälzpumpe (15) als Pumpengehäuse dient, zu welchem Zweck der Sammeltopf (12) und das Pumpengehäuse als ein Bauteil ausgebildet sind, **dadurch gekennzeichnet,**
**dass** die Sprüheinrichtung (23) eine Mehrzahl von um eine gemeinsame Drehachse (9) verdrehbar gelagerten Sprüharmen (7, 8, 28) aufweist,
und **dass** die Umwälzpumpe (15) ein Pumpenrad (17) aufweist, das um die Drehachse (9) der Sprüharme (7, 8, 28) dreht,
wobei die das Pumpenrad (17) tragende Pumpenwelle (18) eine Hohlwelle ist,
die von einer Antriebswelle (20) für die Sprüharme (7, 8, 28) durchragt ist.

2. Automat nach Anspruch 1,
**dadurch gekennzeichnet,**
**dass** die Sprüheinrichtung (23) zur Versorgung mit Spülflotte an ein Versorgungsrohr (24) angeschlossen ist, das sich innerhalb des Spülraums (4) entlang der Drehachse (9) der Sprüharme (7, 8, 28) erstreckt.

3. Automat nach Anspruch 2,
**dadurch gekennzeichnet,**
**dass** das Versorgungsrohr (24) einendseitig an die Umwälzpumpe (15) angeschlossen ist.

4. Automat nach einem der vorhergehenden Ansprüche,
**dadurch gekennzeichnet,**
**dass** das Pumpenrad (17) mit einem stillstehenden Leitrad (21) zusammenwirkt, so dass die vom Pumpenrad (17) geförderte Spülflotte in das Versorgungsrohr (24) gedrückt wird.

5. Automat nach einem der vorhergehenden Ansprüche,
**dadurch gekennzeichnet,**
**dass** das Pumpengehäuse an seiner tiefsten Stelle einen Ablaufstutzen aufweist.

6. Automat nach einem der vorhergehenden Ansprüche 4 bis 5,
**dadurch gekennzeichnet,**
**dass** das Leitrad (21) versorgungsrohrseitig eine Ventileinrichtung (34) aufweist.

## Claims

1. Automatic cleaning and disinfection machine, in particular for medical instruments and appliances, comprising a rinsing container (3), which provides a rinsing chamber (4) and opens into a collecting vessel (12), a spray device (23) arranged in the rinsing chamber (4) for feeding rinsing solution to the washware and a circulating pump (15) which circulates the rinsing solution, the collecting vessel (12) of the circulating pump (15) acting as a pump housing, for which purpose the collecting vessel (12) and the pump housing are formed as one component,
**characterised in that**
the spray device (23) has a plurality of spray arms (7, 8, 28) which are rotatably mounted about a common axis of rotation (9),
and **in that** the circulating pump (15) has a pump wheel (17) which rotates about the axis of rotation (9) of the spray arms (7, 8, 28),
the pump shaft (18) bearing the pump wheel (17) being a hollow shaft through which a drive shaft (20) for the spray arms (7, 8, 28) extends.

2. Automatic machine according to claim 1,
**characterised in that**
the spray device (23) is connected to a supply pipe (24) for supplying rinsing solution, which pipe extends inside the rinsing chamber (4) along the axis of rotation (9) of the spray arms (7, 8, 28).

3. Automatic machine according to claim 2,
**characterised in that**
the supply pipe (24) is connected at one end to the circulating pump (15).

4. Automatic machine according to any of the preceding claims,
**characterised in that**
the pump wheel (17) interacts with a stationary guide wheel (21) so that the rinsing solution conveyed by the pump wheel (17) is pushed into the supply pipe (24).

5. Automatic machine according to any of the preceding claims,
**characterised in that**
the pump housing has a discharge nozzle at its lowest point.

6. Automatic machine according to any of the preceding claims 4 to 5, **characterised in that**
the guide wheel (21) has a valve apparatus (34) on the supply pipe side.

## Revendications

1. Appareil automatique de nettoyage et de désinfection, en particulier pour instruments et appareils médicaux, avec une cuve de lavage (3) fournissant un espace de lavage (4), qui débouche dans un bac collecteur (12), avec un dispositif de pulvérisation (23) disposé dans l'espace de lavage (4) pour l'alimentation des articles à laver en bain de lavage et une pompe de circulation (15) servant à faire circuler le bain de lavage, dans lequel le bac collecteur (12) de la pompe de circulation (15) sert de carter de pompe, et dans ce but le bac collecteur (12) et le carter de pompe sont constitués en tant qu'un composant,
**caractérisé en ce que**
le dispositif de pulvérisation (23) présente une pluralité de bras de pulvérisation (7, 8, 28) supportés de façon à pouvoir tourner autour d'un axe de rotation (9) commun,
et **en ce que** la pompe de circulation (15) présente une roue de pompe (17) qui tourne autour de l'axe de rotation (9) des bras de pulvérisation (7, 8, 28),
dans lequel l'arbre de pompe (18) portant la roue de pompe (17) est un arbre creux qui est traversé par un arbre d'entraînement (20) pour les bras de pulvérisation (7, 8, 28).

2. Appareil automatique selon la revendication 1,
**caractérisé en ce que**
le dispositif de pulvérisation (23) est, pour l'alimentation en bain de lavage, raccordé à un tube d'alimentation (24) qui s'étend à l'intérieur de l'espace de lavage (4) le long de l'axe de rotation (9) des bras de pulvérisation (7, 8, 28).

3. Appareil automatique selon la revendication 2,
**caractérisé en ce que**
le tube d'alimentation (24) est, à une extrémité, raccordé à la pompe de circulation (15).

4. Appareil automatique selon l'une des revendications précédentes,
**caractérisé en ce que**
la roue de pompe (17) coopère avec une roue de guidage (21) fixe de telle sorte que le bain de lavage transporté par la roue de pompe (17) est pressé dans le tube d'alimentation (24).

5. Appareil automatique selon l'une des revendications précédentes,
**caractérisé en ce que**
le carter de pompe présente une tubulure d'évacuation à son emplacement le plus bas.

6. Appareil automatique selon l'une des revendications précédentes 4 à 5, **caractérisé en ce que**
la roue de guidage (21) présente un dispositif de soupape (34) côté tube d'alimentation.
